## Europäisches Patentamt
## European Patent Office
## Office européen des brevets

(19)

(11) Numéro de publication: **0 095 426**
**B1**

(12) ## FASCICULE DE BREVET EUROPEEN

(45) Date de publication du fascicule du brevet:
16.07.86

(51) Int. Cl.⁴: **A 61 K 39/106**, C 07 K 7/10

(21) Numéro de dépôt: **83401052.2**

(22) Date de dépôt: **26.05.83**

(54) **Polypeptides de synthèse contenant au moins une séquence de la sous-unité B1 de la toxine cholérique et médicaments les contenant.**

(30) Priorité: **26.05.82 FR 8209167**

(43) Date de publication de la demande:
**30.11.83 Bulletin 83/48**

(45) Mention de la délivrance du brevet:
**16.07.86 Bulletin 86/29**

(84) Etats contractants désignés:
**BE CH DE FR GB IT LI**

(56) Documents cités:
**FR - A - 2 395 030**
**GB - A - 1 058 828**

**NATURE, vol. 269, no. 5629, 13 octobre 1977, pages 602-604, J. HOLMGREN et al.: "Development of improved cholera vaccine based on subunit toxoid" NATURE, vol. 292, no. 5822, 30 juillet 1981, pages 413-417, MacMillan Journals Ltd., Chesham, Bucks., GB., J. HOLMGREN: "Actions of cholera toxin and the prevention and treatment of cholera"**

(73) Titulaire: **CENTRE NATIONAL DE LA RECHERCHE SCIENTIFIQUE (CNRS), 13 Quai Anatole France, F-75700 Paris (FR)**
Titulaire: **INSTITUT PASTEUR, 25-28, rue du Docteur Roux, F-75724 Paris Cédex 15 (FR)**

(72) Inventeur: **Milhaud, Gérard, 5, Rue des Saints-Pères, F-75006 Paris (FR)**
Inventeur: **Raulais, Daniel, 83, Boulevard Saint-Michel, F-75005 Paris (FR)**
Inventeur: **Rivaille, Pierre, 28, Rue du Petit Musc, F-75004 Paris (FR)**
Inventeur: **Le Thuillier née Tchernov, Georgette, 12, Rue Jean Moulin, F-91420 Moranois (I.P.) (FR)**
Inventeur: **Siffert, Odile, 17, Rue des Missionnaires, F-78000 Versailles (I.P.) (FR)**
Inventeur: **Dodin, André, Courances, F-91490 Milly-la-Foret (FR)**
Inventeur: **Guyon-Gruaz, Anne, 41, Avenue Victor Hugo, F-92100 Boulogne (CNRS) (FR)**
Inventeur: **Delmas, Agnès, 49, Rue Danton, F-94270 Le Kremlin-Bicêtre (FR)**

(74) Mandataire: **Combe, André et al, CABINET BEAU DE LOMENIE 55 rue d'Amsterdam, F-75008 Paris (FR)**

ACTORUM AG.

Dans les polypeptides de l'invention, les acides aminés ont de préférence tous la configuration L.

Les polypeptides de l'invention sont obtenus par synthèse en phase solide avec utilisation des agents protecteurs et des agents de couplage couramment mis en oeuvre dans la synthèse peptidique. La purification des polypeptides selon l'invention peut être réalisée par filtration sur gel et résine échangeuse d'ions. Un exemple de purification est donné dans les exemples illustratifs ci-après.

On a pu montrer que le rôle de ces principes actifs étaient d'une part, en se fixant sur certaines cellules, de pouvoir entrer en compétition avec les autres molécules nocives qui présentent un mode de fixation voisin et d'éviter ladite fixation de ces molécules nocives et, d'autre part, de provoquer la sécrétion de certains anticorps bloquants. Le médicament selon l'invention sera donc actif vis-à-vis de tout microorganisme dont l'action peut être combattue par l'un ou l'autre de ces mécanismes. Il a été trouvé notamment que, de façon tout a fait naturelle, le médicament selon l'invention était utilisable comme médicament curatif ou, de préférence, comme vaccin pour la protection contre le choléra et contre les infections animales ou humaines provenant de l'action d'Escherischia Coli (entérotoxine LT).

Le médicament peut être utilisé par voie orale, intrapéritonéale, sous cutanée ou intraveineuse. Dans le cas d'un vaccin, le nombre total des acides aminés de la séquence sera de préférence de l'ordre de 15 à 30 unités lorsque le peptide est injecté sans porteur naturel ou synthétique d'immunisation.

Les séquences selon l'invention ne sont pas toxiques aux doses normales puisque des souris ont pu recevoir sans présenter des signes graves des doses d'environ 100 microgrammes de produit actif par animal et que des lapins ont pu recevoir avec les mêmes résultats 1 milligramme, par animal, de principe actif.

Les essais pharmacologiques laissent à penser que les médicaments selon l'invention devraient contenir, par prise unitaire, de 50 à 500 mg de principe actif pour un médicament et de 1 à 10 mg de principe actif pour un vaccin.

Les exemples non limitatifs suivants illustrent l'invention:

Exemple 1
Principe de synthèse des séquences selon l'invention:

On ne donne que les principes généraux de la synthèse utilisée puisqu'il s'agit d'une application de méthode connue de synthèse des peptides.
1° support solide
Pour obtenir les polypeptides sous forme de carboxy-terminal libre, on a utilisé, comme support, le chlorométhyl-polystyrène réticulé à 1% avec du divinyl-benzène.
2° Protection des fonctions des acides aminés
La fonction α aminée est protégée par le tertio-butyloxycarbonyle; les fonctions latérales sont protégées par les groupes suivants: Nitro pour le guanidyle de l'Arginine, Ester benzylique pour les β et γ carboxyles des acides aspartique et glutamique, 2-chlorocarbo-benzoxy pour l'ε-amine de la Lysine, benzyl-éther pour les hydroxyles de la Sérine et de la Thréonine, le tosyle pour l'imidazole de l'histidine.
3° Agent de couplage
Chacun des acides aminés est couplé avec un mélange équimoléculaire de dicyclohexylcarbodiimide et de 1-hydroxybenzotriazole, un utilisant comme solvant de couplage le mélange chlorure de méthylène/diméthylformamide (1:1 en volume). La totalité du couplage est contrôlée par un test à la ninhydrine.

Après chaque couplage, on élimine l'agent protecteur de la fonction aminée par traitement à l'aide du mélange acide trifluoroacétique/chlorure de méthylène (30:100 en volume – 30 min) puis neutralisation à l'aide de diisopropyléthylamine dans du chlorure de méthylène (10:90 en volume – 10 min). Après addition de méthionine, on ajoute au mélange acide trifluoroacétique/$CH_2Cl_2$ 1% du mélange réducteur éthane:dithiol (1:2).
4° Coupure du peptide

A la fin de la synthèse le peptide est coupé du polymère par action de l'acide fluorhydrique liquide.
5° Purification
Les peptides sont pourifiés par filtration sur gel. La purification est affinée par chromatographie échangeuse d'ions à l'aide d'un gradient du conductivité.
6° Contrôle de pureté
Elle est effectuée par analyse d'acides aminés après hydrolyse acide et par électrophorèse en milieu sodium dodécylsulfate sur gel de polyacrylamide.

La purification des polypeptides 30–50 et 50–75 a été réalisée selon le mode opératoire ci-après:
A – filtration sur gel
On a utilisé comme gel, le produit connu sous la dénomination commerciale «Biogel P 4» et comme éluant l'acide acétique 1 M. La détection a été effectuée aux UV (à 254 et 280 nm) et par radioactivité, auquel cas on fixe sur le N-terminal du polypeptide synthétisé de la 14 C Alamine.

Les caractéristiques de filtration utilisées pour les deux séquences sont celles indiquées ci-après:
Polypeptide de séquence 30–50
colonne de 100 cm de hauteur, 5 cm de diamètre,
température 20°C
volume de chaque fonction recueillie: 10 ml
vitesse d'élution: 4 fractions à l'heure.

On a recueilli le volume d'élution entre 650–800ml.
Polypeptide de séquence 50–75
colonne de 100 cm de hauteur, 2,5 cm de diamètre
température 4°C
volume de chaque fraction recueillie: 6 ml
vitesse d'élution: 4 fractions à l'heure.
On a recueilli le volume d'élution entre 180–300 ml.

B – Chromatographie sur résine échangeuse d'ions

Les peptides recueillis après lyophilisation des volumes d'élution ont ensuite été chromatographiés sur une colonne de carboxyméthylcellulose CM 32 (diamètre: 2 cm, hauteur: 4 cm) avec comme éluant (gradient linéaire):

1) acétate d'ammonium 1,6 mS pH 4
2) acétate d'ammonium 15,7 mS pH 5,5,
la vitesse d'élution étant de 4 fractions de 5 ml par heure.

La polypeptide 30–50 a élué à 7 mS et le polypeptide 50–75 a élué à 6 mS.

C – Filtration sur gel

On a ensuite effectué une filtration sur Biogel $P_2$® des produits recueillis après chromatographie (colonne de 100 cm de hauteur et 1 cm de diamètre).

L'élution a été effectuée avec de l'acide acétique 1 M (vitesse d'élution: 10,5 ml/h). On a recueilli les fractions entre 87–133 ml.

L'homogénéité des produits ainsi purifiés a été contrôlée par chromatographie en couche mince ($SiO_2$ – éluant: butanol/eau/acide acétique 4:1:1).

Le polypeptide 50–75 a donné un $R_f$ de 0,22 et le polypolypeptide 30–50, un $R_f$ de 0,31.

Par ailleurs, les polypeptides ainsi obtenus ont donné une seule bande sur gel de polyacrylamide à 15% de réticulation (pH 4,3 et pH 8,9).

En chromatolographie liquide haute performance (remplissage: γ Bondapack C 18®), les polypeptides 30–50 et 50–75 ont donné les temps de rétention suivants:

Polypeptide 30–50: temps de rétention 29 min avec le système de solvant ci-après:

A. acide heptafluorobutyrique: eau 0,13:99,87 v/v

B. acide heptafluorobutyrique: n-propanol, 0,13:99,87 v/v

gradient B: 20% à 45% en une heure.

Polypeptide 50–75: temps de rétention 16 mn avec le système de solvants ci-après:

A. acétate de $NH_4$ 0,05 M/$CH_3CN$ (95:5 v/v)

B. acétate de $NH_4$ 0,05 M/$CH_3CN$ (5:95 v/v) gradient B: 20% à 80% en 30 min.

L'analyse des acides aminés des polypeptides 30–50 et 50–75, effectuée par hydrolyse acide en 48 h à 110°C, à l'aide d'acide chlorydrique 6 N + mercapto-éthanol 0,2%, a donné les résultats ci-après:

Polypeptide 30–50

Acide aminé (calculé) trouvé:

Ala(3), 3,09, Arg(1), 0,97, Asp(1), 1,39, Glu(2), 2,43, Gly(2), 2,04, Ile(2), 1,78, Leu(1), 1, Lys(2), 1,86, Met(1), 0,79, Phe(2), 2, Ser(1), 1, Thr(2), 1,99 Val(1), 1,12.

Polypeptide 50–75

Ala(3), 3,09, Arg(2), 2,12, Asp(2), 2,33, Glu(4), 4,17, Gly(1), 1,08, His(1), 0,82, Ile(3), 3,28, Leu(1), 1,06, Lys(3), 3,11, Met(1), 0,85, Ser(2), 1,67, Thr(1), 1,03, Val(1), 1,81.

De la même manière, on a synthétisé le polypeptide constitué par la séquence 30–75 de la sous unité $B_1$ de la toxine cholérique.

Les acides aminés des polypeptides 30–50, 50–75 et 30–75 qui ont été synthétisés comme indiqué ci-dessus avaient tous la configuration L.

Exemple 2

Pour tester les produits synthétisés (séquences 30–50 et 50–75), on a réalisé les essais suivants:

1° Fixation sur les érythrocytes d'homme et de rat La liaison des fragments 30–50, 50–75 et de trois peptides témoins radioactifs a été testée sur les globules rouges. La mesure de la radioactivité libre dans le surnageant ou fixée sur les globules rouges a révélé que le fragment 30–50 se fixe de façon significative et davantage que le fragment 50–75.

2° compétition de fixation avec la toxine totale sur les souches $Y_1$ (cellules de tumeur surrénales de souris)

Le fragment 30–50 utilisé à raison de $10^{-6}$ moles inhibe totalement la production de corticoïdes induites par l'action de la toxine cholérique ($10^{-9}$ molaire) sur ces cellules.

3° Propriétés antigéniques

La séquence 50–75 injectée par voie sous-cutanée à des lapins sans adjuvant ni porteur a entraîné rapidement la production d'anticorps qui procurent au sérum un pouvoir vibriolytique jusqu'à une dilution de 1/360. Cette même séquence injectée 3 fois à 4 jours de distance par voie intrapéritonéale ou buccale à des souris a induit des anticorps reconnaissant la toxine cholérique jusqu'à une dilution supérieure au 1/2000 (test Elisa). Le pouvoir vibriolytique des sérums des souris persiste jusqu'au 1,3000 chez les animaux immunisés par voie buccale et jusqu'au 1/6000 chez ceux immunisés par voie intrapéritonéale.

Tous les animaux immunisés possèdent une bonne protection contre l'action du vibrion cholérique toxinogène et contre la toxine cholérique dans l'épreuve de l'anse ligaturée décrite ci-après:

des souris «$C_{57}$ black» ont été immunisées per os avec de la toxine cholérique (5 μg) ou du polypeptide 50–75 (5 μg) aux jours J, J+10, J+16, J+22 et J+28. Chaque souris a été opérée sous légère anesthésie à l'éther; environ 10 cm de l'iléon ont été ligaturés à 5 cm du pylore et 1 μg de toxine cholérique dans 100 μl de tampon a été injecté (tampon: 0,05 M tris HCl, 0,2 M NaCl, 0,003 M Na $N_3$, 0,001 M $Na_2$ EDTA pH 7,5).

Quatre heures après cette injection les animaux ont été sacrifiés, l'anse a été disséquée et mesurée (L en cm) et pesée (P en g).

Les résultats obtenus sont:

| Toxine cholérique | | | Polypeptide 50–75 | | |
|---|---|---|---|---|---|
| Longueur cm | Poids g | Poids/ Longueur | Longueur cm | Poids g | Poids/ Longueur |
| 9 | 1,72 | 0,191 | 10 | 0,795 | 0,079 |
| 9,5 | 2,23 | 0,234 | 9,5 | 1,505 | 0,158 |
| 9 | 1,895 | 0,210 | 8 | 0,4 | 0,05 |
| 9,5 | 1,76 | 0,185 | 8 | 0,915 | 0,114 |

| Toxine cholérique | | | Polypeptide 50–75 | | |
|---|---|---|---|---|---|
| Longueur cm | Poids g | Poids/ Longueur | Longueur cm | Poids g | Poids/ Longueur |
| 10 | 2,134 | 0,213 | 7 | 0,350 | 0,05 |
| 10,5 | 2,09 | 0,199 | | | |

On a trouvé par ailleurs, que les anticorps antitoxine cholérique ne reconnaissant pas les fragments 30–50 et 50–75, mais que les anticorps anti-polypeptide 30–50 ou 50–75 reconnaissent les séquences qui les ont engendrés, la toxine, les sous unités B et A et l'entérotoxine LT d'Escherichia Coli.

Les résultats ci-dessus ont été mis en évidence par détermination du taux de dilution de sérum reconnaissant encore l'antigène adsorbé sur une plaque Elisa (ledit antigène étant la toxine cholérique, LT Escherichia Coli, la sous unité A et la sous unité B de la toxine cholérique)- Dans ces essais, chaque puits des plaques Elisa et le dosage a été revêtu de 0,25 µg de l'un des antigènes ci-dessus et le dosage a été effectué avec des anticorps de chèvre marqués à la péroxydase, lesdits anticorps de chèvre étant dirigés contre les anticorps totaux de souris.

Les sérums à tester ont été obtenus par immunisation de souris per os ou par voie intrapéritonéale selon le protocole ci-après avec le polypeptide 30–50 ou 50–75:

immunisation per os: une immunisation (2 µg ou 5 µg de polypeptide dans 100 µl du tampon constitué par le mélange 1:1 (en volume) de:

A. bicarbonate de sodium (100 g/l) et de

B. NaCl (8,18 g/l), $PO_4 H Na_2 12 H_2O$) (16,71 g/l) $PO_4 H_2K$ (1,19 g/l)
saigné 8 jours après.

immunisation intrapéritonéale: 2 ou 5 µg de polypeptide dans du sérum physiologique; deux immunisations à 15 jours d'intervalle et rappel un mois après; saignée huit jours après le rappel.

Les résultats obtenus sont consignés dans le tableau ci-après:

| Serum de souris immunisées avec | Antigène (0,25 µg par puits de plaque Elisa) | | | |
|---|---|---|---|---|
| | Sous unité A | Sous unité B | Toxine cholérique | Lt Escherichia coli |
| 30–50 per os | 1/2000 | ≃0 | 1/600 | 1/800 |
| 30–50 i.p. | 1/500 | 1/80 | 1/400 | 1/300 |
| 50–75 per os | 1/1500 | 1/120 | 1/900 | 1/600 |
| 50/75 i.p. | 1/1200 | 1/160 | 1/300 | 1/300 |

## Revendications

1. Médicaments caractérisés en ce qu'ils comportent au moins une séquence de la sous unité $B_1$ de la toxine cholérique, ladite séquence comportant au moins une arginine en 35, 67 ou 73 de ladite sous unité et étant choisie parmi les séquences 30–50, 50–75 et 30–75 de ladite sous unité.

2. Médicaments selon la revendication 1, caractérisés en ce qu'ils contiennent de 50 à 500 mg de ladite séquence.

3. Médicament selon l'une des revendications 1 ou 2, caractérisé en ce qu'il est un vaccin.

4. A titre de nouveau produit, le polypeptide constitué par au moins une séquence de la sous unité $B_1$ de la toxine cholérique, ladite séquence comportant au moins une arginine en 35, 67 ou 73 de ladite sous unité et étant choisie parmi les séquences 30–50, 50–75 et 30–75 de ladite sous-unité.

5. Polypeptide selon la revendication 4, caractérisé en ce qu'il est constitué par la séquence 30–50:

H₂N.Ser - Leu - Ala - Gly - Lys - Arg - Glu - Met - Ala
30                                    35

- Ile - Ile - Thr - Phe - Lys - Asn - Gly - Ala - Thr - Phe
40                                    45

- Glu - Val - COOH.
50

6. Polypeptide selon la revendication 4, caractérisé en ce qu'il est constitué par la séquence 50–75:

H₂N.Val - Glu - Val - Pro - Gly - Ser - Gln - His - Ile -
50                                    55

Asp - Ser - Gln - Lys - Lys - Ala - Ile - Glu - Arg - Met
60                                    65

- Lys - Asn - Thr - Leu - Arg - Ile - Ala - COOH.
70                                    75

7. Polypeptide selon la revendication 4, caractérisé en ce qu'il est constitué par la séquence 30–75:

H₂N.Ser - Leu - Ala - Gly - Lys - Arg - Glu - Met - Ala
30                                    35

- Ile - Ile - Thr - Phe - Lys - Asn - Gly - Ala - Thr - Phe
40                                    45

- Glu - Val - Glu - Val - Pro - Gly - Ser - Gln - His - Ile
50                                    55

- Asp - Ser - Gln - Lys - Lys - Ala - Ile - Glu - Arg -
60                                    65

Met - Lys - Asn - Thr - Leu - Arg - Ile - Ala - COOH.
70                                    75

## Patentansprüche

1. Medikamente, dadurch gekennzeichnet, dass sie mindestens eine Sequenz der Untereinheit $B_1$ des Choleratoxins enthalten, welche Sequenz mindestens ein Arginin in Position 35, 67 oder 73 der Untereinheit aufweist und ausgewählt ist aus den Sequenzen 30 bis 50, 50 bis 75 und 30 bis 75 der Untereinheit.

2. Medikamente nach Anspruch 1, dadurch gekennzeichnet, dass sie 50 bis 500 mg der Sequenz enthalten.

3. Medikament nach einem der Ansprüche 1 oder 2, dadurch gekennzeichnet, dass es eine Vakzine ist.

4. Als neues Produkt das Polypeptid, das durch mindestens eine Sequenz der Untereinheit B₁ des Chloeratoxins gebildet ist, welche Sequenz mindestens ein Arginin in Position 35, 67 oder 73 der Untereinheit aufweist und ausgewählt ist aus den Sequenzen 30 bis 50, 50 bis 75 und 30 bis 75 der Untereinheit.

5. Polypeptid nach Anspruch 4, dadurch gekennzeichnet, dass es aus der Sequenz 30 bis 50:

H₂N.Ser - Leu - Ala - Gly - Lys - Arg - Glu - Met - Ala
30 35

- Ile - Ile - Thr - Phe - Lys - Asn - Gly - Ala - Thr - Phe
40 45

- Glu - Val - COOH
50

besteht.

6. Polypeptid nach Anspruch 4, dadurch gekennzeichnet, dass es aus der Sequenz 50 bis 75:

H₂N.Val - Glu - Val - Pro - Gly - Ser - Gln - His - Ile -
50 55

Asp - Ser - Gln - Lys - Lys - Ala - Ile - Glu - Arg - Met
60 65

- Lys - Asn - Thr - Leu - Arg - Ile - Ala - COOH.
70 75

besteht.

7. Polypeptid nach Anspruch 4, dadurch gekennzeichnet, dass es aus der Sequenz 30 bis 75:

H₂N.Ser - Leu - Ala - Gly - Lys - Arg - Glu - Met - Ala
30 35

- Ile - Ile - Thr - Phe - Lys - Asn - Gly - Ala - Thr - Phe
40 45

- Glu - Val - Glu - Val - Pro - Gly - Ser - Gln - His - Ile
50 55

- Asp - Ser - Gln - Lys - Lys - Ala - Ile - Glu - Arg -
60 65

Met - Lys - Asn - Thr - Leu - Arg - Ile - Ala - COOH
70 75

besteht.

**Claims**

1. Drugs characterized in that they comprise at least one sequence of B₁ subunit of cholera toxin, said sequence comprising at least one arginine in 35, 67 or 73 of said subunit and being selected from sequences 30–50, 50–75 and 30–75 of said subunit.

2. Drugs according to claim 1, characterized in that they contain 50 to 500 mg of said sequence.

3. Drugs according to any one of claims 1 or 2, characterized in that it is a vaccine.

4. As novel product, the polypeptide constituted by at least one sequence of B₁ subunit of cholera toxin, said sequence comprising at least one arginine in 35, 67 or 73 of said subunit and being selected from sequences 30–50, 50–75 and 30–75 of said subunit.

5. Polypeptide according to claim 4, characterized in that it is constituted by the 30–50 sequence:

H₂N.Ser - Leu - Ala - Gly - Lys - Arg - Glu - Met - Ala
30 35

- Ile - Ile - Thr - Phe - Lys - Asn - Gly - Ala - Thr - Phe
40 45

- Glu - Val - COOH
50

6. Polypeptide according to claim 4, characterized in that it is constituted by the 50–75 sequence:

H₂N.Val - Glu - Val - Pro - Gly - Ser - Gln - His - Ile -
50 55

Asp - Ser - Gln - Lys - Lys - Ala - Ile - Glu - Arg - Met
60 65

- Lys - Asn - Thr - Leu - Arg - Ile - Ala - COOH.
70 75

7. Polypeptide according to claim 4, characterized in that it is constituted by the 30–75 sequence:

H₂N.Ser - Leu - Ala - Gly - Lys - Arg - Glu - Met - Ala
30 35

- Ile - Ile - Thr - Phe - Lys - Asn - Gly - Ala - Thr - Phe
40 45

- Glu - Val - Glu - Val - Pro - Gly - Ser - Gln - His - Ile
50 55

- Asp - Ser - Gln - Lys - Lys - Ala - Ile - Glu - Arg -
60 65

Met - Lys - Asn - Thr - Leu - Arg - Ile - Ala - COOH.
70 75